(19) Europäisches Patentamt
European Patent Office
Office européen des brevets

(11) EP 4 293 028 A1

(12) **EUROPEAN PATENT APPLICATION**
published in accordance with Art. 153(4) EPC

(43) Date of publication:
**20.12.2023 Bulletin 2023/51**

(21) Application number: **22752311.5**

(22) Date of filing: **10.02.2022**

(51) International Patent Classification (IPC):
***C07D 519/00*** (2006.01) ***A61K 31/407*** (2006.01)
***A61P 7/02*** (2006.01) ***A61P 9/10*** (2006.01)

(52) Cooperative Patent Classification (CPC):
**A61K 31/407; A61P 7/02; A61P 9/10; C07D 519/00**

(86) International application number:
**PCT/CN2022/075740**

(87) International publication number:
**WO 2022/171151 (18.08.2022 Gazette 2022/33)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **10.02.2021 CN 202110184080**
**10.12.2021 CN 202111503546**
**31.12.2021 CN 202111664079**

(71) Applicants:
- **Shanghai Senhui Medicine Co., Ltd.**
  **Shanghai 201203 (CN)**
- **Shanghai Shengdi Pharmaceutical Co., Ltd**
  **Shanghai 201210 (CN)**
- **Jiangsu Hengrui Pharmaceuticals Co., Ltd.**
  **Lianyungang, Jiangsu 222047 (CN)**

(72) Inventors:
- **ZHU, Lingjian**
  **Shanghai 201203 (CN)**
- **YU, Xiuzhao**
  **Shanghai 201203 (CN)**
- **LIU, Xiaowu**
  **Shanghai 201203 (CN)**
- **BEN, Peipei**
  **Lianyungang, Jiangsu 222047 (CN)**
- **CHEN, Lei**
  **Lianyungang, Jiangsu 222047 (CN)**
- **KONG, Xianglin**
  **Lianyungang, Jiangsu 222047 (CN)**
- **HUANG, Jian**
  **Shanghai 201203 (CN)**
- **SUN, Piaoyang**
  **Lianyungang, Jiangsu 222047 (CN)**

(74) Representative: **Regimbeau**
**20, rue de Chazelles**
**75847 Paris Cedex 17 (FR)**

(54) **SMTP-7 DERIVATIVE AND USE THEREOF**

(57) The present disclosure relates to an SMTP-7 derivative and the use thereof. In particular, provided is a compound as represented by formula I or a pharmaceutically acceptable salt thereof, wherein each group is as defined in the description.

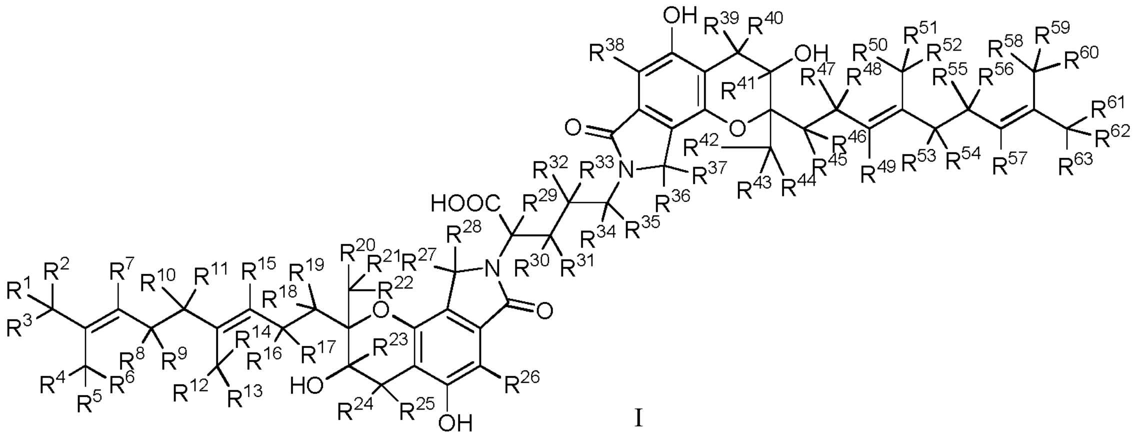

I

## Description

### TECHNICAL FIELD

The disclosure belongs to the field of pharmaceutics, and relates to an SMTP-7 derivative and use thereof.

### BACKGROUND

SMTP-7 (TMS-007, *Stachybotrys microspore* triprenyl phenol-7) was extracted from a mold (*Stachybotrys microspora*) on a special kind of fallen leaves on Iriomote Island of Okinawa prefecture in 2000. It is a small-molecule plasminogen activator and is similar to vitamin E in structure. It breaks down blood clots through a novel mechanism of action and is also believed to be capable of inhibiting local inflammation at the site of a thrombus. In addition, SMTP-7 has anti-tumor angiogenesis activity, anti-oxidation activity and pro-tissue regeneration activity (WEIMIN H, SHIGEKI O, et al. J. Antibiot., 2000, 53(3): 241-247).

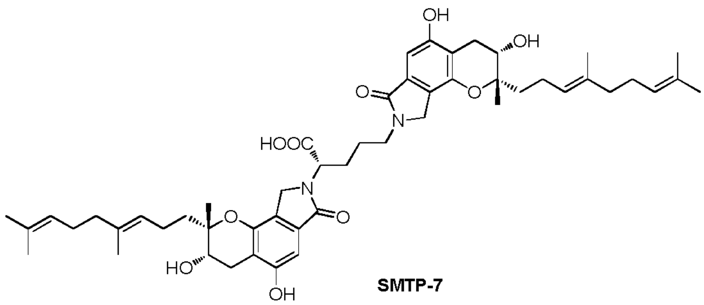

SMTP-7

Plasminogen is a plasmin precursor, which can be activated to produce plasmin. This is a protease, which can hydrolyze many proteins, including thrombospondin. Upon binding to plasminogen, SMTP-7 changes its molecular conformation so that it is more easily activated by the plasminogen activator. Therefore, SMTP-7 per se does not have the function of activating plasminogen but makes the activation process easier. The unique combined action of SMTP-7 makes it possible for SMTP-7 to become a best-in-class thrombolytic drug for treating acute ischemic stroke (AIS). Compared to the existing standard thrombolytic drugs, SMTP-7 has the potential to prolong the therapeutic window (while many antihypertensive, lipid-lowering and anticoagulant drugs can prevent strokes, the only therapeutic drug is the recombinant tissue plasminogen activator (rt-PA, alteplase) for ischemic strokes, whose major ingredient is a glycoprotein containing 526 amino acids).

The SMTP molecule induces a change in the conformation of plasminogen, resulting in accelerated binding of plasminogen to fibrin and finally activation leading to plasmin. In addition, SMTP induces plasmin to self-cleave to provide angiogenic human angiostatin-like fragments. This activity is believed to be the mechanism of the anti-angiogenesis and anti-tumor effects of SMTP molecules. In addition, SMTP-induced increases in activated plasminogen may control local extracellular proteolysis, thereby leading to tissue remodeling, wound healing and tissue regeneration.

### SUMMARY

The disclosure provides a compound of formula I or a pharmaceutically acceptable salt thereof,

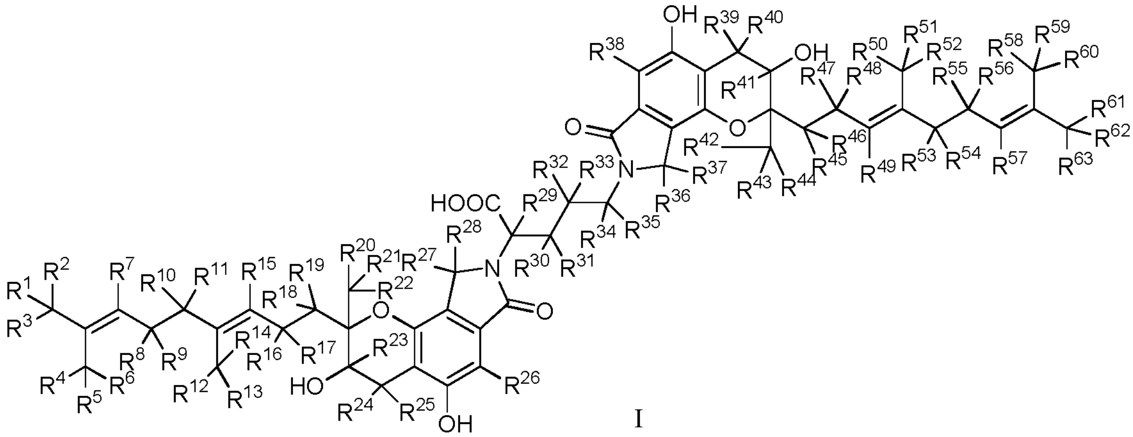

I

wherein $R^1$, $R^2$, $R^3$, $R^4$, $R^5$, $R^6$, $R^7$, $R^8$, $R^9$, $R^{10}$, $R^{11}$, $R^{12}$, $R^{13}$, $R^{14}$, $R^{15}$, $R^{16}$, $R^{17}$, $R^{18}$, $R^{19}$, $R^{20}$, $R^{21}$, $R^{22}$, $R^{23}$, $R^{24}$, $R^{25}$, $R^{26}$, $R^{27}$, $R^{28}$, $R^{29}$, $R^{30}$, $R^{31}$, $R^{32}$, $R^{33}$, $R^{34}$, $R^{35}$, $R^{36}$, $R^{37}$, $R^{38}$, $R^{39}$, $R^{40}$, $R^{41}$, $R^{42}$, $R^{43}$, $R^{44}$, $R^{45}$, $R^{46}$, $R^{47}$, $R^{48}$, $R^{49}$, $R^{50}$, $R^{51}$, $R^{52}$, $R^{53}$, $R^{54}$, $R^{55}$, $R^{56}$, $R^{57}$, $R^{58}$, $R^{59}$, $R^{60}$, $R^{61}$, $R^{62}$ and $R^{63}$ are each independently hydrogen or deuterium, and at least one of $R^1$, $R^2$, $R^3$, $R^4$, $R^5$, $R^6$, $R^7$, $R^8$, $R^9$, $R^{10}$, $R^{11}$, $R^{12}$, $R^{13}$, $R^{14}$, $R^{15}$, $R^{16}$, $R^{17}$, $R^{18}$, $R^{19}$, $R^{20}$, $R^{21}$, $R^{22}$, $R^{23}$, $R^{24}$, $R^{25}$, $R^{26}$, $R^{27}$, $R^{28}$, $R^{29}$, $R^{30}$, $R^{31}$, $R^{32}$, $R^{33}$, $R^{34}$, $R^{33}$, $R^{36}$, $R^{37}$, $R^{38}$, $R^{39}$, $R^{40}$, $R^{41}$, $R^{42}$, $R^{43}$, $R^{44}$, $R^{45}$, $R^{46}$, $R^{47}$, $R^{48}$, $R^{49}$, $R^{50}$, $R^{51}$, $R^{52}$, $R^{53}$, $R^{54}$, $R^{55}$, $R^{56}$, $R^{57}$, $R^{58}$, $R^{59}$, $R^{60}$, $R^{61}$, $R^{62}$ and $R^{63}$ is deuterium.

In some embodiments, in the compound of formula I or the pharmaceutically acceptable salt thereof, $R^{27}$ is selected from deuterium; $R^{28}$ is selected from deuterium; $R^{36}$ is selected from deuterium; $R^{37}$ is selected from deuterium.

In some embodiments, in the compound of formula I or the pharmaceutically acceptable salt thereof, $R^{26}$ is selected from deuterium; $R^{38}$ is selected from deuterium.

In some embodiments, in the compound of formula I or the pharmaceutically acceptable salt thereof, $R^{24}$ is selected from deuterium; $R^{25}$ is selected from deuterium; $R^{39}$ is selected from deuterium; $R^{40}$ is selected from deuterium.

In some embodiments, in the compound of formula I or the pharmaceutically acceptable salt thereof, $R^{29}$ is selected from deuterium.

In some embodiments, in the compound of formula I or the pharmaceutically acceptable salt thereof, $R^{30}$ is selected from deuterium; $R^{31}$ is selected from deuterium.

In some embodiments, in the compound of formula I or the pharmaceutically acceptable salt thereof, $R^{34}$ is selected from deuterium; $R^{35}$ is selected from deuterium.

In some embodiments, in the compound of formula I or the pharmaceutically acceptable salt thereof, $R^{16}$ is selected from deuterium; $R^{17}$ is selected from deuterium; $R^{47}$ is selected from deuterium; $R^{48}$ is selected from deuterium.

In some embodiments, in the compound of formula I or the pharmaceutically acceptable salt thereof, $R^{12}$ is selected from deuterium; $R^{13}$ is selected from deuterium; $R^{14}$ is selected from deuterium.

In some embodiments, in the compound of formula I or the pharmaceutically acceptable salt thereof, $R^{50}$ is selected from deuterium; $R^{51}$ is selected from deuterium; $R^{52}$ is selected from deuterium.

In some embodiments, in the compound of formula I or the pharmaceutically acceptable salt thereof, $R^{23}$ is selected from deuterium; $R^{41}$ is selected from deuterium.

In some embodiments, in the compound of formula I or the pharmaceutically acceptable salt thereof, $R^{32}$ is selected from deuterium; $R^{33}$ is selected from deuterium.

In some embodiments, in the compound of formula I or the pharmaceutically acceptable salt thereof, $R^{30}$ is selected from deuterium; $R^{31}$ is selected from deuterium; $R^{32}$ is selected from deuterium; $R^{33}$ is selected from deuterium; $R^{34}$ is selected from deuterium; $R^{35}$ is selected from deuterium.

In some embodiments, in the compound of formula I or the pharmaceutically acceptable salt thereof, $R^{29}$ is selected from deuterium; $R^{30}$ is selected from deuterium; $R^{31}$ is selected from deuterium; $R^{32}$ is selected from deuterium; $R^{33}$ is selected from deuterium; $R^{34}$ is selected from deuterium; $R^{35}$ is selected from deuterium.

In some embodiments, in the compound of formula I or the pharmaceutically acceptable salt thereof, $R^1$ is selected from deuterium; $R^2$ is selected from deuterium; $R^3$ is selected from deuterium.

In some embodiments, in the compound of formula I or the pharmaceutically acceptable salt thereof, $R^4$ is selected from deuterium; $R^5$ is selected from deuterium; $R^6$ is selected from deuterium.

In some embodiments, in the compound of formula I or the pharmaceutically acceptable salt thereof, $R^{58}$ is selected from deuterium; $R^{59}$ is selected from deuterium; $R^{60}$ is selected from deuterium.

In some embodiments, in the compound of formula I or the pharmaceutically acceptable salt thereof, $R^{61}$ is selected from deuterium; $R^{62}$ is selected from deuterium; $R^{63}$ is selected from deuterium.

In some embodiments, in the compound of formula I or the pharmaceutically acceptable salt thereof, $R^1$ is

selected from deuterium; $R^2$ is selected from deuterium; $R^3$ is selected from deuterium; $R^4$ is selected from deuterium; $R^5$ is selected from deuterium; $R^6$ is selected from deuterium; $R^{58}$ is selected from deuterium; $R^{59}$ is selected from deuterium; $R^{60}$ is selected from deuterium; $R^{61}$ is selected from deuterium; $R^{62}$ is selected from deuterium; $R^{63}$ is selected from deuterium.

In some embodiments, in the compound of formula I or the pharmaceutically acceptable salt thereof, $R^7$ is selected from deuterium; $R^{57}$ is selected from deuterium.

In another aspect, the compound of formula I or the pharmaceutically acceptable salt thereof provided in some embodiments is

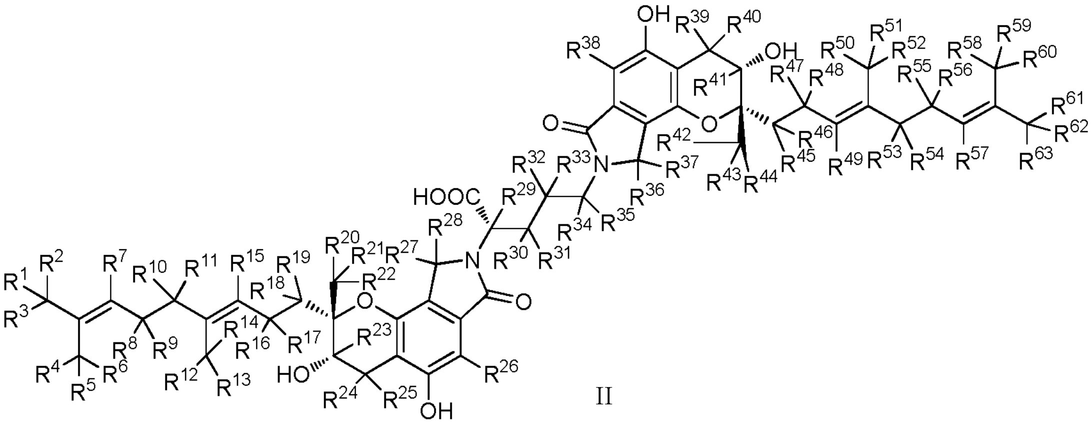

II

.

Typical compounds of formula I include, but are not limited to:

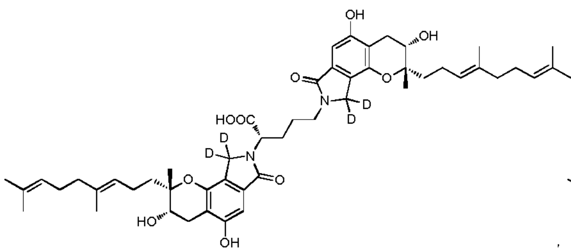

,

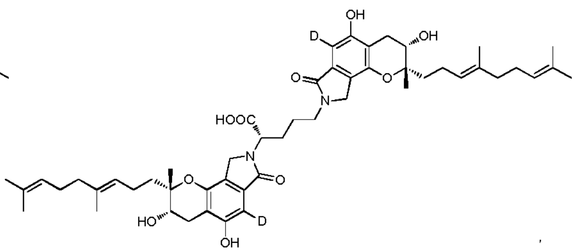

,

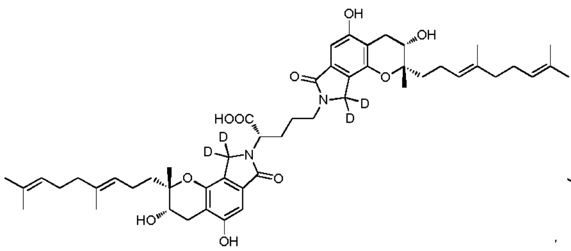

,

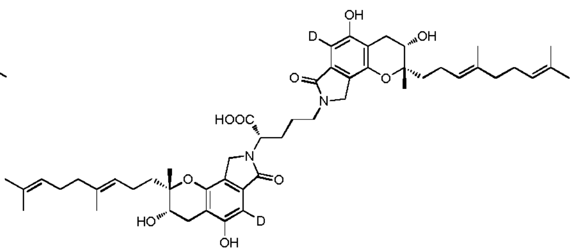

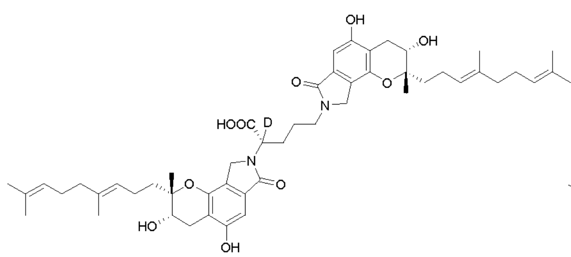

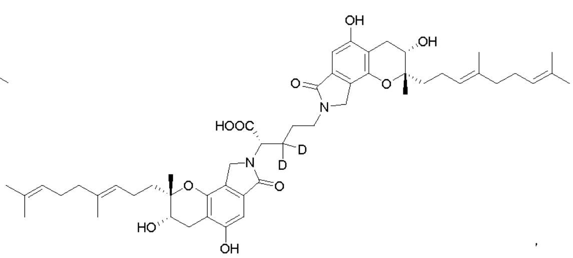

,

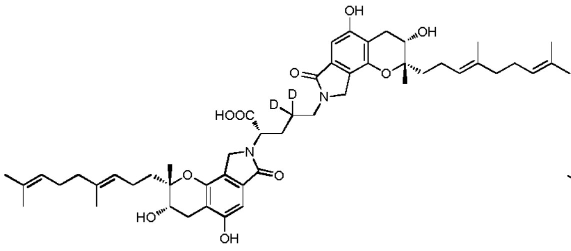
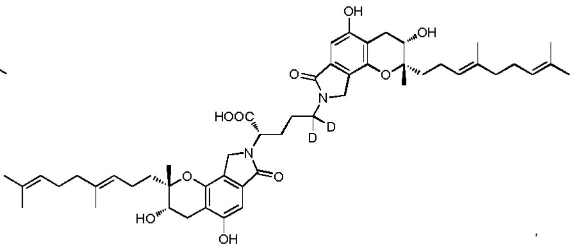,
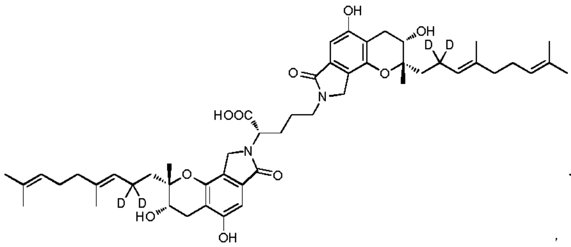,
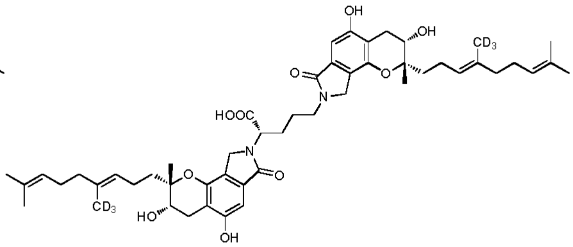
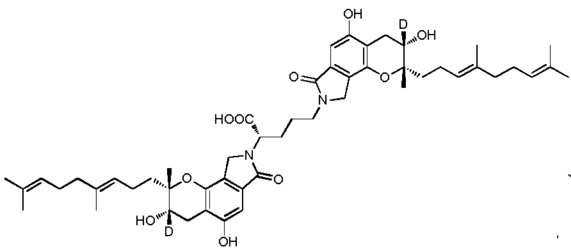,
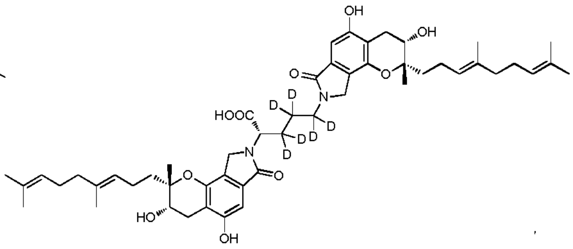,
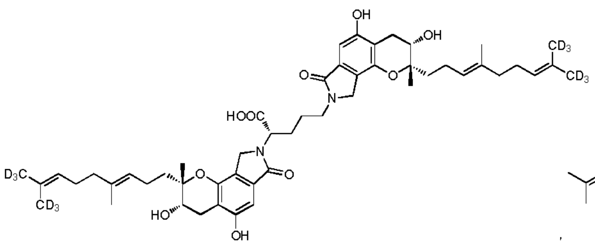,
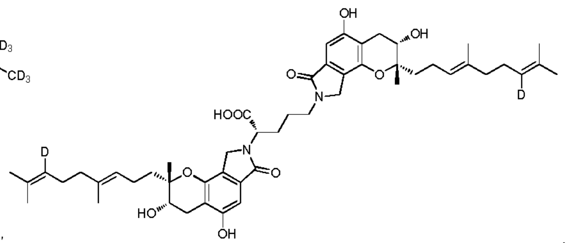.

The disclosure also provides a pharmaceutical composition comprising a therapeutically effective amount of at least one of the compounds of formula I or the pharmaceutically acceptable salts thereof described above and a pharmaceutically acceptable excipient.

In some embodiments, a unit dose of the pharmaceutical composition is 0.001 mg-1000 mg.

In certain embodiments, the pharmaceutical composition comprises 0.01-99.99% of the compound of formula (I) or the pharmaceutically acceptable salt thereof described above on the basis of the total weight of the composition. In certain embodiments, the pharmaceutical composition comprises 0.1-99.9% of the compound of formula I or the pharmaceutically acceptable salt thereof described above. In certain embodiments, the pharmaceutical composition comprises 0.5%-99.5% of the compound of formula I or the pharmaceutically acceptable salt thereof described above. In certain embodiments, the pharmaceutical composition comprises 1%-99% of the compound of formula I or the pharmaceutically acceptable salt thereof described above. In certain embodiments, the pharmaceutical composition comprises 2%-98% of the compound of formula I or the pharmaceutically acceptable salt thereof described above.

In certain embodiments, the pharmaceutical composition comprises 0.01%-99.99% of a pharmaceutically acceptable excipient based on the total weight of the composition. In certain embodiments, the pharmaceutical composition comprises 0.1%-99.9% of a pharmaceutically acceptable excipient. In certain embodiments, the pharmaceutical composition comprises 0.5%-99.5% of a pharmaceutically acceptable excipient. In certain embodiments, the pharmaceutical composition comprises 1%-99% of a pharmaceutically acceptable excipient. In certain embodiments, the pharmaceutical composition comprises 2%-98% of a pharmaceutically acceptable excipient.

The disclosure also provides a method for preventing and/or treating a cardiovascular or cerebrovascular disease, which comprises administering to a patient a therapeutically effective amount of the compound of formula I or the pharmaceutically acceptable salt thereof described above. In some embodiments, the disease is selected from a

thromboembolic disease. In some embodiments, the disease is selected from the group consisting of myocardial infarction, angina pectoris, reocclusion and restenosis after angioplasty or aortic coronary artery shunt, disseminated intravascular coagulation, stroke, transient ischemic attack, peripheral arterial occlusive disease, pulmonary embolism and deep vein thrombosis.

The disclosure also provides use of the compound of formula I or the pharmaceutically acceptable salt thereof described above or the pharmaceutical composition described above in the preparation of a medicament for preventing and/or treating a cardiovascular or cerebrovascular disease. In some embodiments, the disease is selected from a thromboembolic disease. In some embodiments, the disease is selected from the group consisting of myocardial infarction, angina pectoris, reocclusion and restenosis after angioplasty or aortic coronary artery shunt, disseminated intravascular coagulation, stroke, transient ischemic attack, peripheral arterial occlusive disease, pulmonary embolism and deep vein thrombosis.

The disclosure also provides the compound of formula I or the pharmaceutically acceptable salt thereof described above for preventing and/or treating a cardiovascular or cerebrovascular disease. In some embodiments, the disease is selected from a thromboembolic disease. In some embodiments, the disease is selected from the group consisting of myocardial infarction, angina pectoris, reocclusion and restenosis after angioplasty or aortic coronary artery shunt, disseminated intravascular coagulation, stroke, transient ischemic attack, peripheral arterial occlusive disease, pulmonary embolism and deep vein thrombosis.

In another aspect, the pharmaceutically acceptable salt of the compound in the disclosure is selected from the group consisting of an inorganic salt and an organic salt.

In another aspect, the compounds of the disclosure may exist in specific geometric or stereoisomeric forms. The disclosure contemplates all such compounds, including cis and trans isomers, (-)- and (+)-enantiomers, (R)- and (S)-enantiomers, diastereomers, (D)-isomer, (L)-isomer, and racemic mixtures and other mixtures thereof, such as enantiomerically or diastereomerically enriched mixtures, all of which are within the scope of the disclosure. Additional asymmetric carbon atoms may be present in substituents such as an alkyl group. All such isomers and mixtures thereof are included within the scope of the disclosure.

Optically active (R)- and (S)-enantiomers, and D- and L-isomers can be prepared by chiral synthesis, chiral reagents or other conventional techniques. If one enantiomer of a certain compound of the disclosure is desired, it may be prepared by asymmetric synthesis or derivatization with a chiral auxiliary, wherein the resulting mixture of diastereomers is separated and the auxiliary group is cleaved to provide the pure desired enantiomer. Alternatively, when the molecule contains a basic functional group (e.g., amino) or an acidic functional group (e.g., carboxyl), salts of diastereomers are formed with an appropriate optically active acid or base, followed by resolution of diastereomers by conventional methods known in the art, and the pure enantiomers are obtained by recovery. In addition, separation of enantiomers and diastereomers is generally accomplished by chromatography using a chiral stationary phase, optionally in combination with chemical derivatization (e.g., carbamate formation from amines).

In the chemical structure of the compound of the disclosure, a bond "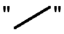" represents an unspecified configuration-that is, if chiral isomers exist in the chemical structure, the bond "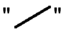" may be "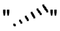" or "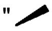", or contains both the configurations of "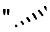" and "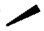". In the chemical structure of the compound of the disclosure, a bond "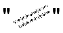" is not specified with a configuration, that is, it may be in a Z configuration or an E configuration, or contains both configurations.

The compounds and intermediates of the disclosure may also exist in different tautomeric forms, and all such forms are included within the scope of the disclosure. The term "tautomer" or "tautomeric form" refers to structural isomers of different energies that can interconvert via a low energy barrier. For example, proton tautomers (also known as proton transfer tautomers) include interconversion via proton migration, such as keto-enol and imine-enamine, lactam-lactim isomerization. An example of a lactam-lactim equilibrium is present between A and B as shown below.

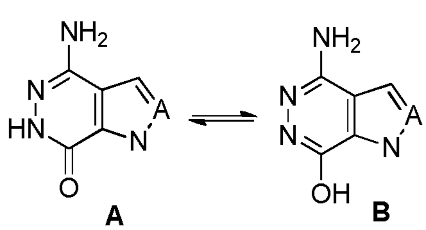

A B

All compounds in the disclosure can be drawn as form A or form B. All tautomeric forms are within the scope of the disclosure. The nomenclature of the compounds does not exclude any tautomers.

The disclosure also includes isotopically-labeled compounds which are identical to those recited herein but have one or more atoms replaced by an atom having an atomic mass or mass number different from the atomic mass or mass number usually found in nature. Examples of isotopes that can be incorporated into the compound of the

disclosure include isotopes of hydrogen, carbon, nitrogen, oxygen, phosphorus, sulfur, fluorine, iodine, and chlorine, such as $^{2}H$, $^{3}H$, $^{11}C$, $^{13}C$, $^{14}C$, $^{13}N$, $^{15}N$, $^{15}O$, $^{17}O$, $^{18}O$, $^{31}P$, $^{32}P$, $^{35}S$ , $^{18}F$, $^{123}I$, $^{125}I$ and $^{36}Cl$.

Unless otherwise stated, when a position is specifically designated as deuterium (D), that position shall be understood to be deuterium having an abundance that is at least 3000 times greater than the natural abundance of deuterium (which is 0.015%) (i.e., incorporating at least 45% deuterium). In certain embodiments, the abundance of each of the specified deuterium atoms in the compound of the disclosure is at least 3500 times (52.5% deuterium incorporation at each of the specified deuterium atoms), at least 4000 times (60% deuterium incorporation), at least 4500 times (67.5% deuterium incorporation), at least 5000 times (75% deuterium incorporation), at least 5500 times (82.5% deuterium incorporation), at least 6000 times (90% deuterium incorporation), at least 6333.3 times (95% deuterium incorporation), at least 6466.7 times (97% deuterium incorporation), at least 6600 times (99% deuterium incorporation) or at least 6633.3 times (99.5% deuterium incorporation) the natural abundance of deuterium. The disclosure also includes various deuterated forms of the compound of formula (I). Each available hydrogen atom connected to a carbon atom may be independently replaced by a deuterium atom. Those skilled in the art are able to synthesize the deuterated forms of the compound of formula (I) with reference to the relevant literature. Commercially available deuterated starting materials can be used in preparing the deuterated forms of the compound of formula (I), or they can be synthesized using conventional techniques with deuterated reagents including, but not limited to, deuterated borane, tri-deuterated borane in tetrahydrofuran, deuterated lithium aluminum hydride, deuterated iodoethane, deuterated iodomethane, and the like.

"Pharmaceutical composition" refers to a mixture containing one or more of the compounds or the physiologically or pharmaceutically acceptable salts or pro-drugs thereof described herein, and other chemical components, for example, physiologically or pharmaceutically acceptable carriers and excipients. The pharmaceutical composition is intended to promote the administration to an organism, so as to facilitate the absorption of the active ingredient, thereby exerting biological activity.

"Pharmaceutically acceptable excipient" or "acceptable excipient" includes, but is not limited to, any adjuvant, carrier, excipient, glidant, sweetener, diluent, preservative, dye/colorant, flavoring agent, surfactant, wetting agent, dispersant, suspending agent, stabilizer, isotonic agent, solvent or emulsifier that has been approved by the U.S. food and drug administration as acceptable for use in humans or livestock animals.

"Effective amount" or "therapeutically effective amount" described herein includes an amount sufficient to ameliorate or prevent a symptom or condition of a medical condition. An effective amount also refers to an amount sufficient to allow or facilitate diagnosis. The effective amount for a particular patient or veterinary subject may vary with factors such as the condition to be treated, the general health of the patient, the method and route and dosage of administration, and the severity of side effects. An effective amount may be the maximum dose or administration regimen to avoid significant side effects or toxic effects.

## DETAILED DESCRIPTION

The disclosure is further described below with reference to examples, which are not intended to limit the scope of the disclosure.

Experimental procedures without conditions specified in the examples of the disclosure were generally conducted according to conventional conditions, or according to conditions recommended by the manufacturers of the starting materials or commercial products. Reagents without origins specified are commercially available conventional reagents.

The structures of the compounds were determined by nuclear magnetic resonance (NMR) spectroscopy and/or mass spectrometry (MS). NMR shifts ($\delta$) are given in $10^{-6}$ (ppm). NMR analysis was performed on a Bruker AVANCE-400 nuclear magnetic resonance instrument, with deuterated dimethyl sulfoxide (DMSO-$d_6$), deuterated chloroform ($CDCl_3$) and deuterated methanol ($CD_3OD$) as solvents and tetramethylsilane (TMS) as an internal standard.

MS analysis was performed on an Agilent 1200/1290 DAD-6110/6120 Quadrupole MS liquid chromatography-mass spectrometry system (manufacturer: Agilent; MS model: 6110/6120 Quadrupole MS),
waters ACQuity UPLC-QD/SQD (manufacturer: waters, MS model: waters ACQuity Qda Detector/waters SQ Detector), and THERMO Ultimate 3000-Q Exactive (manufacturer: THERMO, MS model: THERMO Q Exactive).

High performance liquid chromatography (HPLC) analysis was performed using the following HPLC instruments: Agilent HPLC 1200DAD, Agilent HPLC 1200VWD and Waters HPLC e2695-2489.

Chiral HPLC analysis was performed on an Agilent 1260 DAD high performance liquid chromatograph.

Preparative high performance liquid chromatography used Waters 2545-2767, Waters 2767-SQ Detecor2, Shimadzu LC-20AP and Gilson GX-281 preparative chromatographs.

Preparative chiral chromatography used a Shimadzu LC-20AP preparative chromatograph.

The CombiFlash preparative flash chromatograph used was CombiFlash Rf200 (TELEDYNE ISCO).

Yantai Huanghai HSGF254 or Qingdao GF254 silica gel plates, 0.15-0.2 mm layer thickness, were adopted

for thin-layer chromatography (TLC) analysis and 0.4-0.5 mm layer thickness for TLC separation and purification.

Silica gel column chromatography generally used 200- to 300-mesh silica gel (Huanghai, Yantai) as the carrier.

Known starting materials described herein may be synthesized using or according to methods known in the art, or may be purchased from ABCR GmbH & Co. KG, Acros Organics, Aldrich Chemical Company, Accela ChemBio Inc., Chembee Chemicals, and other companies.

In the examples, the reactions can all be performed in an argon atmosphere or a nitrogen atmosphere unless otherwise specified.

The argon atmosphere or nitrogen atmosphere means that the reaction flask is connected to a balloon containing about 1 L of argon or nitrogen.

The hydrogen atmosphere means that the reaction flask is connected to a balloon containing about 1 L of hydrogen.

Pressurized hydrogenation reactions were performed using a Parr 3916EKX hydrogenator and a Qinglan QL-500 hydrogenator, or an HC2-SS hydrogenator.

Hydrogenation reactions generally involve 3 cycles of vacuumization and hydrogen purging.

Microwave reactions were performed on a CEM Discover-S 908860 microwave reactor.

In the examples, a solution refers to an aqueous solution unless otherwise specified.

In the examples, reactions were conducted at room temperature, i.e., 20 °C to 30 °C, unless otherwise specified.

The monitoring of the reaction progress in the examples was conducted by thin-layer chromatography (TLC). The developing solvent for reactions, the eluent system for column chromatography purification of compounds, the developing solvent system for thin-layer chromatography and the volume ratio of the solvents were adjusted according to the polarity of the compound, or by adding a small amount of basic or acidic reagents such as triethylamine and acetic acid.

**Example 1: Preparation of SMTP-7**

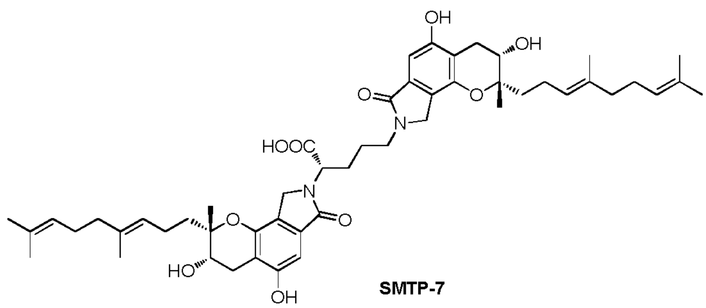

**SMTP-7**

*Stachybotrys microspora* IFO30018 was inoculated into a seed medium (4% glucose, 0.5% soybean meal, 0.3% dry broth, 0.3% yeast extract, 0.01% antifoaming agent, pH 5.8) and cultured for 4 days. The seed culture liquid was inoculated into a fermentation medium (5% sucrose, 0.1% yeast extract, 0.3% NaNO3, 0.1% $K_2HPO_4$, 0.05% $MgSO_4{\cdot}7H_2O$, 0.05% KCl, 0.00025% $CoCl_2{\cdot}6H_2O$, 0.0015% $FeSO_4{\cdot}7H_2O$, 0.00065% $CaCl_2{\cdot}2H_2O$, 0.01% antifoaming agent, pH 5.8). After 4 days of culture, L-ornithine was added; the culture was continued for 1 day, and fermentation was complete.

The fermentation broth was extracted with methanol. The extract was concentrated by rotary evaporation and extracted with ethyl acetate. The extract was dehydrated with anhydrous sodium sulfate, filtered, concentrated, dried and solidified.

The solid was dissolved in methanol, and the solution was subjected to pre-treatment and preparative purification using reversed-phase packing material. After steps such as ethyl acetate extraction, the target product was obtained.

**Example 2: Preparation of δ-Deutero-L-ornithine (**Compound **1e)**

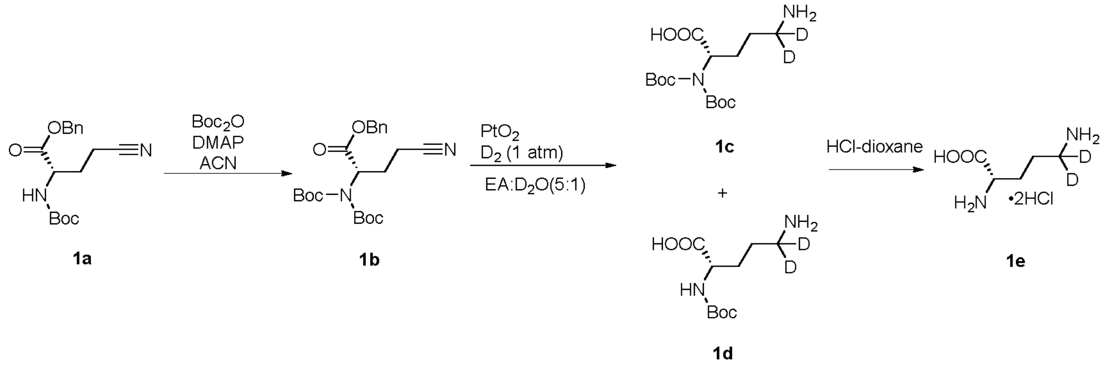


1a 1b 1c 1d 1e

**Benzyl (S)-2-(bis(tert-butoxycarbonyl)amino)-4-cyanobutanoate (**Compound **1b)**

Compound **1a** (prepared according to Synlett, 2016, vol. 27, 2, 309-312; 74.8 g, 234.9 mmol) was dissolved in acetonitrile (750 mL), $Boc_2O$ (76.9 g, 352.4 mmol) was added, and DMAP (2.9 g, 23.5 mmol) was added. Then the reaction was conducted at 45 °C for 1-1.5 h. The reaction mixture was concentrated by rotary evaporation to remove the solvent. The crude product was purified by column chromatography to give the target compound **2b** (95 g, 99.4% purity, 100% yield); MS (ESI) m/z 441.2 $[M+Na]^+$.

**Compounds 1c and 1d**

Compound **1b** (4.18 g, 10 mmol) was dissolved in EA (100 mL) (super dry) and $D_2O$ (20 mL), and $PtO_2$ (204 mg) was added. The system was purged with $D_2$, and the reaction was conducted in a $D_2$ atmosphere at 30 °C (external temperature) for 40 h. The reaction was substantially complete. The reaction mixture was separated, and the aqueous phase was washed with EA and then directly lyophilized to give a solid (1.87 g). The solid was dissolved in acetonitrile (5 mL) and ethyl acetate (20 mL). The solution was stirred at room temperature and filtered. The filter cake was washed with ethyl acetate and dried using an oil pump to give a mixture of compounds **1c** and **1d** (1.54 g, 46% yield).

**δ-Deutero-L-ornithine (**Compound **1e)**

The mixture of compounds **1c** and **1d** (195 mg, 0.58 mmol) was dissolved in a 6 M aqueous solution of hydrochloric acid, and the reaction was conducted at room temperature for 2 h. The reaction was substantially complete. The reaction mixture was concentrated by rotary evaporation to remove the solvent. The residue was dried to a constant weight using an oil pump to give a pale yellow solid product, compound **1e** (120 mg, 100% yield, 97.25% purity).

HNMR ($D_2O$, 400M): 1.95-1.94 (m, 4H), 2.91-2.98 (m, 0.027H), 3.96 (t, J = 6.4 Hz, 1H).

**Example 3: Preparation of δ-Deutero SMTP-7 (**Compound **1)**

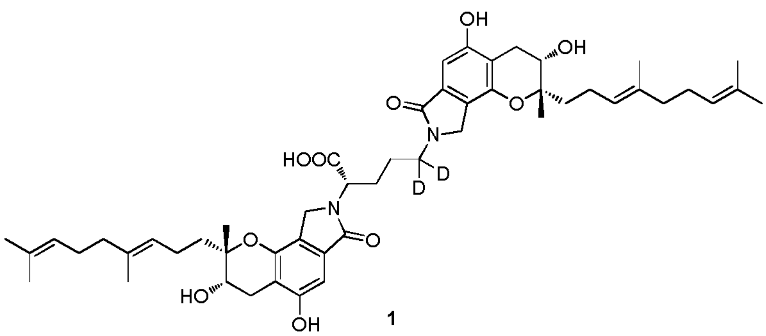

1

Compound **1** was prepared using δ-deutero L-ornithine (prepared according to Example 2) according to the method of Example 1.

*Stachybotrys microspora* IFO30018 was inoculated into a seed medium (4% glucose, 0.5% soybean meal, 0.3% dry broth, 0.3% yeast extract, 0.01% antifoaming agent, pH 5.8) and cultured for 4 days. The seed culture liquid

was inoculated into a fermentation medium (5% sucrose, 0.1% yeast extract, 0.3% NaNO3, 0.1% $K_2HPO_4$, 0.05% $MgSO_4 \cdot 7H_2O$, 0.05% KCl, 0.00025% $CoCl_2 \cdot 6H_2O$, 0.0015% $FeSO_4 \cdot 7H_2O$, 0.00065% $CaCl_2 \cdot 2H_2O$, 0.01% antifoaming agent, pH 5.8). After 4 days of culture, δ-deutero L-ornithine was added; the culture was continued for 1 day, and fermentation was complete.

The fermentation broth was extracted with methanol. The extract was concentrated by rotary evaporation and extracted with ethyl acetate. The extract was dehydrated with anhydrous sodium sulfate, filtered, concentrated, dried and solidified.

The solid was dissolved in methanol, and the solution was subjected to pre-treatment and preparative purification using reversed-phase packing material. After steps such as ethyl acetate extraction, the target product was obtained.

$^1$H NMR (400 MHz, DMSO-*d6*) δ 13.07-12.66 (br., 1H), 9.79 (s, 1H), 9.73 (s, 1H), 6.66 (s, 1H), 6.62 (s, 1H), 5.28-5.09 (m, 3H), 5.07-4.95 (m, 2H), 4.72 (dd, J = 9.9, 5.7 Hz, 1H), 4.26-4.05 (m, 4H), 3.73 (dd, J = 13.0, 6.9 Hz, 2H), 2.82 (dt, J = 17.0, 4.8 Hz, 2H), 2.48-2.38 (m, 2H), 2.17-2.05 (m, 4H), 2.04-1.95 (m, 4H), 1.95-1.81 (m, 6H), 1.66-1.46 (m, 23H), 1.18 (s, 3H), 1.15 (s, 3H).

Test Example 1: Pharmacokinetic Study in Rats

1.1. Preparation of test samples

A proper amount of each of SMTP-7 and compound 1 was weighed out, and 2% (final volume) DMSO and 98% (final volume) normal saline were added. After they were well mixed by vortexing and ultrasonication, a 1 mg/mL clear solution was obtained for later use.

1.2. Animals

SD rats, at the age of 6-8 weeks, weighing about 180-220 g.

1.3. Regimen

| Group | Number of rats/sex | Test sample | Dose (mg/kg) | Concentration of test compound (mg/mL) | Route of administration |
|---|---|---|---|---|---|
| 1 | 3 rats/ male | SMTP-7 | 5 | 1 | Intravenous injection |
| 2 | 3 rats/ male | Compound 1 | 5 | 1 | Intravenous injection |

1.4. Sample collection

About 0.20 mL of blood was collected for each sample via the jugular vein or other suitable routes, and sodium heparin was used as the anticoagulant. The blood was placed on ice immediately after the collection. The collection was performed at a total of 10 time points: before administration, and 5 min, 0.25 h, 0.5 h, 1 h, 2 h, 4 h, 6 h, 10 h and 24 h after administration. The collected blood samples were placed in heparin anticoagulant blood collection tubes and centrifuged at 6800 g at 2-8 °C for 6 min to separate plasma. The plasma samples were stored in a freezer at -80 °C before analysis.

1.5. Biological analysis and data processing

The plasma concentration of each test compound was determined. The analysis of quality control samples was performed while the samples were analyzed, and more than 66.7% of the quality control samples were required to have an accuracy of 80-120%. When plasma drug concentration-time curves were plotted, BLQ was recorded as 0. When the pharmacokinetic parameters were calculated, the concentration before administration was calculated as 0; BLQ before Cmax (including "No peak") was calculated as 0; and BLQ that occurs after $C_{max}$ (including "No peak") was excluded from the calculation. The following pharmacokinetic parameters were calculated from the plasma concentration data of different time points using the non-compartmental analysis method of Phoenix WinNonlin 7.0 software: $AUC_{(0-t)}$, $AUC_{(0-\infty)}$, $T_{1/2}$, MRT, $C_{max}$, $T_{max}$, etc.

Experimental results:

| Group | $T_{1/2}$ (h) | $T_{max}$ (h) | $C_{max}$ (ng/ml) | $AUC_{(0-t)}$ (h*ng/ml) | $MRT_{(0-t)}$ |
|---|---|---|---|---|---|
| 1 | 6.689±0.178 | 0.08±0.00 | 26,311.98±6,236.41 | 8,009.467±1,832.168 | 0.465±0.080 |
| 2 | 8.94±0.61 | 0.08±0.00 | 19,078.40±4,312.75 | 11,287.80±1,377.70 | 3.06±0.25 |

Conclusion: Compound 1 showed a longer half-life and a lower $C_{max}$ than SMTP-7 after intravenous administration to SD rats.

**Example 4: Preparation of α-Deutero-L-ornithine**

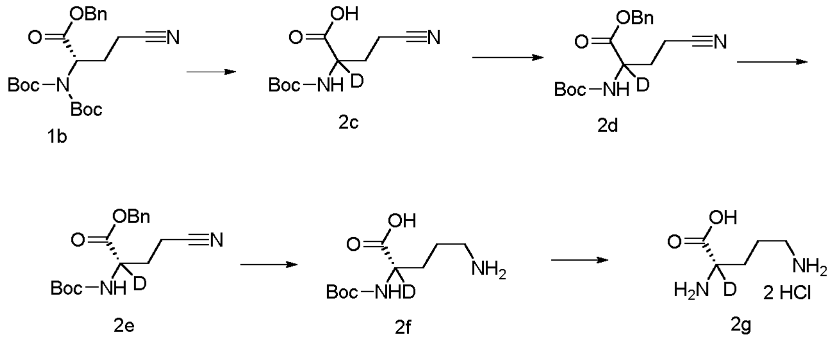

1b 2c 2d 2e 2f 2g

**α-Deutero-2-((tert-butoxycarbonyt)amino)-4-cyanobutyric acid (** Compound **2c)**

In a nitrogen atmosphere, compound **1b** (prepared according to Example 1; 25.0 g, 59.8 mmol) was added to 125 mL of MeOD and dissolved by stirring, and anhydrous potassium carbonate (41.3 g, 299.0 mmol) was added. The reaction was conducted at 20-30 °C for 16 h. The reaction was substantially complete. The system was directly concentrated to give a crude compound **2c.** MS (ESI) m/z 252.1 $[M+Na]^+$.

**Benzyl α-deutero-2-((tert-butoxycarbonyt)amino)-4-cyanobutanoate (**Compound **2d)**

To the crude compound **2c** (25.0 g, 59.8 mmol) was added 150 mL of anhydrous acetonitrile followed by BnBr (15.3 g, 89.7 mmol). The reaction was conducted in a nitrogen atmosphere at 20-30 °C for 6 h. The reaction was substantially complete. The reaction mixture was filtered. The filter cake was washed with acetonitrile. The filtrate was concentrated, and the crude product was purified by column chromatography to give the target compound **2d** (6.8 g), which was identified by chiral HPLC as a racemate (35.7% yield over two steps); MS (ESI) m/z 343.1 [M+Na]+.

**Benzyl α-deutero-(S)-2-((tert-butoxycarbonyl)amino)-4-cyanobutanoate (**Compound **2e)**

The above-prepared racemic product, compound **2d** (6.8 g), was chirally resolved to give compound **2e** (3.6 g).
Instrument: MGII preparative SFC (SFC-14); chiral column: ChiralPak AY, 250 × 30 mm I.D., 5 μm; mobile phase A: carbon dioxide; mobile phase B: methanol (0.1% ammonia water), gradient 15%; flow rate: 60 mL/min; back pressure: 100 bar; column temperature: 38 °C; detection wavelength: 220 nm; length of separation: about 6 min.
HNMR (CDCl3, 400M): 1.44 (s, 9H), 1.58-1.65 (m,1H), 1.97-2.04 (m, 1H), 2.23-2.28 (m, 1H),, 2.38-2.46 (m, 1H), 5.17-5.20 (m, 2H), 7.36-7.38 (m, 5H).

**α-Deutero-(S)-5-amino-2-((tert-butoxycarbonyt)amino)pentanoic acid (**Compound **2f)**

Compound **2e** (3.5 g, 10.9 mmol) was weighed out and dissolved in 70 mL of ethyl acetate, 700 mL of purified water was added, and $PtO_2$ (224 mg, 0.9 mmol) was added.
The system was purged with hydrogen, and the reaction was conducted at 20-30 °C for 16 h. The reaction was

substantially complete. The reaction mixture was filtered, and the filtrate was separated. The aqueous phase was collected, washed with water and lyophilized to give a crude product, the target compound **2f** (about 1.6 g). The crude product was triturated with 20 mL of ethyl acetate and 2 mL of acetonitrile, and the triturate was filtered. The solid was dried under reduced pressure to give a purified product (about 1.5 g, 58.6% yield). MS-ESI: m/z 236.1 [M+H]+.

HNMR (D2O, 400M):1.44 (s, 9H), 1.58-1.80 (m,4H), 2.93-2.98 (m, 2H).

**α-Deutero-L-ornithine (**Compound **2g)**

Compound **2f** (1.5 g, 6.4 mmol) was weighed out and dissolved in about 10 mL of 6 M HCl by stirring. The reaction was conducted at 20-30 °C for 2 h. The reaction was substantially complete. The reaction mixture was directly lyophilized to give a crude compound **2g** (1.2 g). The crude compound was triturated with 20 mL of acetonitrile, and the triturate was filtered to give a solid purified product (1.1 g, 83.9% yield). MS-ESI: m/z 134.1 [M+H]+.

HNMR (D2O, 400M): 1.67-2.01 (m,4H), 2.98-3.01 (m, 2H).

**Example 5: Preparation of α-Deutero SMTP-7 (**Compound **2)**

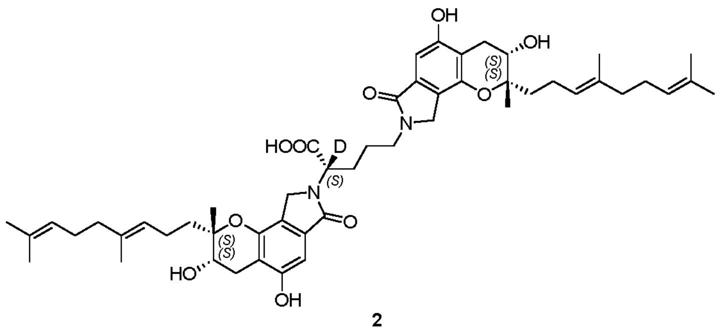

**2**

The target product, compound **2,** was prepared using α-deutero L-ornithine (prepared according to Example 4) according to the method of Example 1.

**Example 6: Preparation of β-Deutero-L-ornithine (**Compound **3e)**

**β-Deutero-L-ornithine (**Compound **3e)**

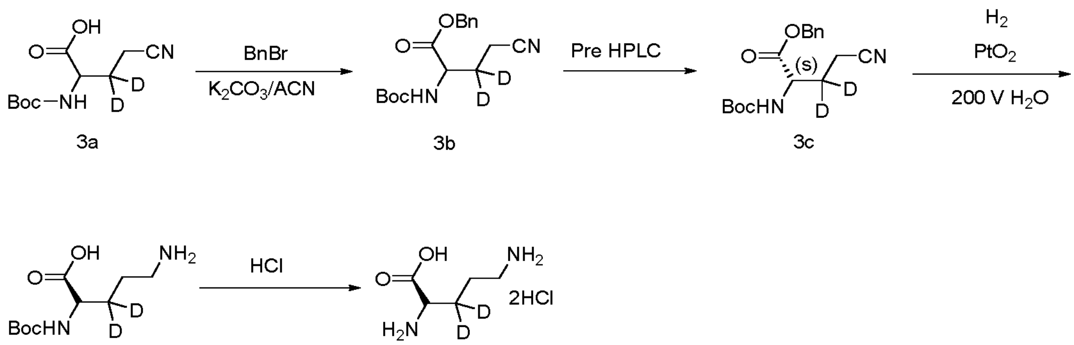


3a 3b 3c 3d 3e

## **Compound 3b**

Compound **3a** (3.8 g, 15.4 mmol, prepared according to Journal of the American Chemical Society, 2017, vol. 139, 39, 13830-13836) and anhydrous potassium carbonate (10.6 g, 77 mmol) were dissolved in 60 mL of anhydrous acetonitrile, and BnBr (5.3 g, 30.8 mmol) was added. The reaction was conducted at 10-20 °C for 16 h.

The reaction mixture was filtered to remove insoluble matter. The organic phase was washed with EA. The

filtrate was concentrated to give a crude product. The crude product was purified by column chromatography (PE:EA = 10:1) to give the target compound **3b** (4.8 g, 93.3% purity, 97% yield).

MS-ESI: m/z 343.1 $[M+Na]^+$
$^1$HNMR ($CDCl_3$, 400M):1.45 (s, 9H), 4.36-4.40 (m,1H), 5.17-5.24 (s, 2H), 7.27-7.40 (m, 5H).

**Compound 3c**

Compound **3b** was a racemate. It was purified by preparative chiral chromatography to give a configuration monomer, the target compound **3c** (2.2 g).

**Compound 3d**

Compound **3c** (2.2 g, 6.9 mmol) was dissolved in EA and water by stirring, and PtO2 (0.3 g, 1.3 mmol) was added. The system was purged with hydrogen three times, and the reaction was conducted at 10-20 °C for 16 h. The reaction mixture was filtered, and about 50 mL of EA was added to the aqueous phase. The mixture was separated, and the aqueous phase was collected, concentrated under reduced pressure and dried using an oil pump to give a crude product. The crude product was triturated at 10-20 °C with 20 mL of ethyl acetate and 2 mL of acetonitrile, and the triturate was filtered. The solid was washed with EA, collected and dried to give the target compound **3d** (1.2 g).
MS-ESI: m/z 235.1 $[M+H]^+$

**Compound 3e**

Compound **3d** (1.2 g, 5.1 mmol) was dissolved in 12 mL of a 6 M aqueous solution of hydrochloric acid. The reaction was conducted at 10-20 °C for 4 h. The reaction mixture was directly concentrated under reduced pressure using an oil pump to give the target compound **3e** (1.05 g, 100% yield).

MS-ESI: m/z 135.1 $[M+H]^+$
$^1$HNMR ($CDCl_3$, 400M):1.67-1.81 (m, 2H), 1.91-1.93 (m,0.1H), 2.95-2.32 (m, 2H), 3.98 (s, 1H).

**Example 7: Preparation of β-Deutero SMTP-7 (**Compound **3)**

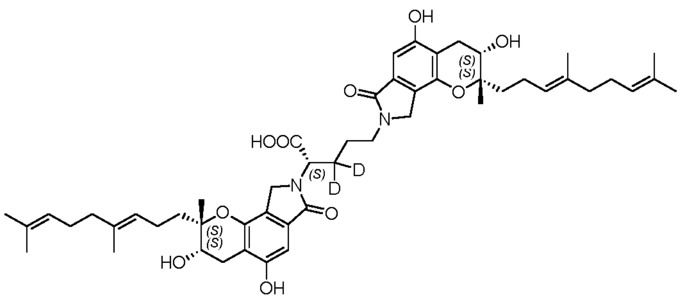

**3**

The target product, compound 3, was prepared using β-deutero L-ornithine (prepared according to Example 6) according to the method of Example 1.

**Example 8: Preparation of γ-Deutero-L-ornithine (**Compound **4c)**

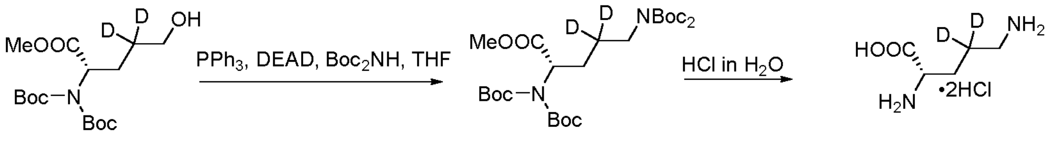


4a 4b 4c

## Compound 4b

Compound **4a** (2.2 g, 6.4 mmol, prepared according to Journal of the American Chemical Society, 2018, vol. 140, 23, 7116-7126) was dissolved in tetrahydrofuran (50 mL), and Boc$_2$NH (2.1 g, 9.6 mmol) and triphenylphosphine (5.0 g, 19.1 mmol) were added. The mixture was cooled to 0 °C, and DEAD (3.3 g, 19.1 mmol) was slowly added dropwise. After the addition, the reaction was warmed to room temperature and stirred overnight and was quenched with phosphate buffer. The reaction mixture was extracted with ethyl acetate. The organic phases were combined, washed with saturated brine, concentrated and purified by column chromatography (EA:PE = 1:20) to give the target product, compound **4b** (1.7 g, 50% yield).

$^1$H NMR (500 MHz, $CDCl_3$) δ 4.87 (dd, J= 9.5, 5.0 Hz, 1H), 3.70 (s, 3H), 3.59 (d, J= 3.1 Hz, 2H), 2.09 (dd, J= 14.2, 4.7 Hz, 1H), 1.88-1.81 (m, 1H), 1.49 (d, J= 3.3 Hz, 36H).

**Compound 4c**

Compound **4b** (1.7 g, 3.2 mmol) and a 4 M aqueous solution of hydrochloric acid (30 mL) were added to a 100 mL sealed tube. The reaction was heated to 90 °C (external temperature) and was conducted for 16 h. The reaction mixture was cooled to room temperature, then concentrated and dried using an oil pump to give compound **4c** (500 mg, 92% yield).

MS-ESI: m/z 135.1 $[M+H]^+$
$^1$H NMR (500 MHz, D2O) δ 3.99 (t, J= 6.3 Hz, 1H), 3.03 (s, 2H), 1.97 (qd, J= 14.5, 6.5 Hz, 2H).

**Example 9: Preparation of γ-Deutero SMTP-7 (**Compound **4)**

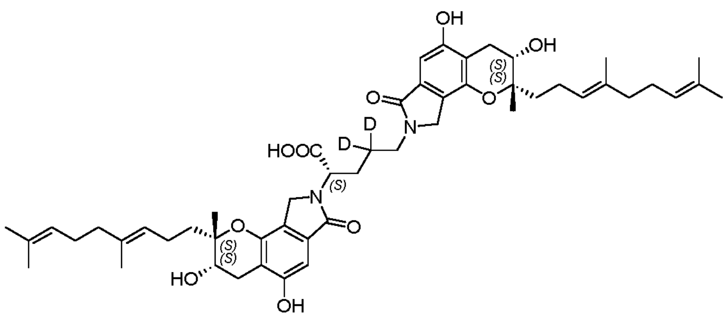

4

The target product, compound **4,** was prepared using γ-deutero L-ornithine (prepared according to Example 8) according to the method of Example 1.

Test Example 2: Pharmacokinetic Study in Rats

1.1. Preparation of test samples

A proper amount of each of SMTP-7, compound 2, compound 3 and compound 4 was weighed out, and normal saline was added (the pH was adjusted to about 9.2 with a 2 mM NaOH solution). After they were well mixed by vortexing and ultrasonication, a 1 mg/mL clear solution was obtained for later use.

1.2. Animals

SD rats, at the age of 6-8 weeks, weighing about 180-240 g.

1.3. Regimen

| Group | Test sample | Dose (mg/kg) | Concentration of test compound (mg/mL) | Route of administration |
|---|---|---|---|---|
| 1 | SMTP-7 | 5 | 1 | Intravenous injection |
| 2 | Compound 2 | 5 | 1 | Intravenous injection |
| 3 | Compound 3 | 5 | 1 | Intravenous injection |
| 4 | Compound 4 | 5 | 1 | Intravenous injection |

The pharmacokinetic parameters such as $AUC_{(0-t)}$, $AUC_{(0-\infty)}$, $T_{1/2}$, MRT, $C_{max}$ and $T_{max}$ were measured in rats according to the method of Test Example 1.

Experimental results: After intravenous administration to rats, SMTP-7, compound 2 and compound 3 did not greatly differ in $T_{1/2}$ and AUC; compound 4 showed a lower $T_{1/2}$ and a lower AUC than SMTP-7.

Test Example 3: Thromboembolism in Rats

1.1. Preparation of a cerebral infarction model

An embolic stroke model was prepared as the cerebral infarction model according to the method in the literature (J Cereb Blood Flow Metab, 1997, 17(2):123-135). 0.1 mL of rat blood was collected and immediately sucked into a PE50 catheter. The catheter was left at room temperature for 2 h and then stored at 4 °C for 22 h. The thrombus was pushed into 30 mL of normal saline and washed 3 times, for 5 min each time. A 5-mm length of embolus was cut out and sucked into a PE50 catheter with a special end for later use.

A rat was anesthetized with isoflurane and then fixed to an operating table in a supine position. A cut was made in the skin along the median line of the neck. The right common carotid artery was isolated, the internal carotid artery branch was isolated, and the pterygopalatine was clamped using an artery clip. A small cut was made in the common carotid artery, and the above embolus in the catheter was pushed into the cranium with 0.4 mL of normal saline. The catheter was carefully withdrawn. The common carotid artery was ligated, and the cut in the skin was sutured.

2.2. Grouping and administration

Neurological function scoring was performed 1 h after the model was established. A score of ≥8 indicates that the model was successfully established. The rats were divided into a sham surgery group, a model control group, a test drug group (5, 10 and 20 mg/kg) and a control drug group (10 mg/kg). Each group included 10 rats. The sham surgery group and the model control group were administered normal saline. The test drug group was administered compound 1 (1 mg/mL, prepared in normal saline (the pH was adjusted to about 9.2 with a 2 mM NaOH solution)). The control drug group was administered SMTP-7 (1 mg/mL, prepared in normal saline (the pH was adjusted to about 9.2 with a 2 mM NaOH solution)). Intravenous administration was immediately performed 1 h after the model was established. 10% of the drug liquid was first injected, and the remaining 90% was infused over 30 min. The experiment ended 24 h after the administration.

**Neurological function scoring**

The extent of behavioral disorders in the animals was observed and scored before administration and 24 h after treatment. The scoring criteria were as follows:

Criteria for scoring neurological function damage in MCAO rats

| Test | | Performance | Score |
|---|---|---|---|
| 1. Motion testing | 1) Lift the tail of the rat | | |
| | | Flexion of the forelimbs | 1 |
| | | Flexion of the hindlimbs | 1 |
| | | Holds up its head within 30 s > 10. (with the vertical axis) | 1 |
| | 2) Allow the rat to walk on the floor (normal = 0; maximum = 3) | | |
| | | 0. Normally walks | 0 |
| | | 1. Cannot walk straight | 1 |
| | | 2. Circles to the hemiplegia side | 2 |
| | | 3. Falls to the hemiplegia side | 3 |
| 2. Balance testing on a horizontal bar | | Can balance in a stable posture | 0 |
| | | Grasps the lateral sides of the horizontal bar | 1 |
| | | Can hold the horizontal bar, but one limb falls off from the horizontal bar | 2 |
| | | Can hold the horizontal bar, but two limbs fall off from the horizontal bar | 3 |
| | | Or swings on the horizontal bar for over 60 s | |
| | | Attempts to balance on the horizontal bar (>40 s), but falls off | 4 |
| | | Attempts to balance on the horizontal bar (>20 s), but falls off | 5 |
| | | Falls off, fails to balance, or does not want to struggle and hangs from the horizontal bar | 6 |
| 3. Reflex testing | | Auricular reflex (shakes its head when the entrance of the ear canal is touched) | 1 |
| | | Corneal reflex (blinks its eyes when the cornea is gently touched with cotton) | 1 |
| | | Startle reflex (produces a motion response when hearing the transient sound of tearing paper) | 1 |
| | | Seizures, myoclonus, dystonia | 1 |
| Total | | | 16 |

**Cerebral hemorrhage assay**

24 h after the scoring and blood collection, cardiac perfusion was performed and the brain was collected. The brain tissue was frozen in a -20 °C freezer and then sectioned from front to back; each section was 2 mm thick. A section scored 1 point if hemorrhage was present. The sum of the scores of the sections was the total hemorrhage score of each animal.

**Cerebral infarction range assay**

24 h after the scoring and blood collection, cardiac perfusion was performed and the brain was collected. The brain tissue was frozen in a -20 °C freezer and then sectioned from front to back; each section was 2 mm thick. The brain tissue sections were placed into a 2% solution of tetrazolium chloride (TTC) and incubated at 37 °C for 5 min. The infarcted tissues were white, and the non-infarcted tissues were red. The cerebral infarction area was measured using Image J software, and the percentage of the infarction area to the total brain area was calculated.

$$\text{Percentage of cerebral infarction area\%} = \text{cerebral infarction area} / \text{total brain area} \times 100\%$$

**Data analysis method**

The metrology data are expressed as $\bar{x}\pm s$, and pairwise comparisons were made using T-TEST; P<0.05

indicates a statistical difference.

**Experimental results:**

Table 1: Behavioral scores and cerebral infarction area

| Group | Dose (mg/kg) | n | Behavioral scores | | Infarction area% |
|---|---|---|---|---|---|
| | | | Before administration | 24 h after administration | |
| Sham surgery | Normal saline | 10 | 0±0 | 0±0 | 0±0 |
| Model group | Normal saline | 15 | 10.4±0.5### | 8.4±2.1### | 6.07±4.98### |
| | 5 | 10 | 10.5±0.5 | 7.1±1.8 | 2.47±2.21* |
| Compound 1 | 10 | 10 | 10.2±0.4 | 6.5±2.5* | 1.38±0.9** |
| | 20 | 10 | 10.2±0.4 | 6.6±2.2* | 1.7±1.1* |
| SMTP-7 | 10 | 10 | 10.2±0.4 | 6.3±2.4* | 1.9±2.35* |

Note: # means it is relative to the sham surgery group; * means it is relative to the model group.

Table 2: Cerebral hemorrhage scores

| Group | Dose (mg/kg) | n | Hemorrhage scores | |
|---|---|---|---|---|
| | | | ≥1 (probability) | ≥3 (probability) |
| Sham surgery | Normal saline | 10 | 0 (0%) | 0 (0%) |
| Model group | Normal saline | 15 | 4 (27%) | 0 (0%) |
| | 5 | 10 | 2 (20%) | 0 (0%) |
| Compound 1 | 10 | 10 | 1 (10%) | 0 (0%) |
| | 20 | 10 | 1 (10%) | 0 (0%) |
| SMTP-7 | 10 | 10 | 2 (20%) | 1 (10%) |

**In terms of behavior,** when administered at the same dose (10 mg/kg), both compound 1 and SMTP-7 showed improvements compared to the model group and the improvements were comparable.

**In terms of cerebral infarction area,** both compound 1 and SMTP-7 showed significant improvements compared to the model group, and compound 1 was superior to SMTP-7 (a reduction of about 37%) when administered at the same dose of 10 mg/kg; the probability of serious infarction was relatively low.

**In terms of cerebral hemorrhage,** each dose group of compound 1 and SMTP-7 showed an improvement compared to the model group; the probability of cerebral hemorrhage after compound 1 (10%) was administered at the dose of 10 mg/kg was lower than that after SMTP-7 (20%) was administered at the dose of 10 mg/kg, and in addition, there was a cerebral hemorrhage score of ≥3 (10%) after SMTP-7 administration, which suggests that after SMTP-7 administration, there is a higher risk of hemorrhagic conversion in the brain, which is unfavorable for the clinical treatment of ischemic cerebral stroke.

**Conclusion:** Compound 1 has an improving effect on both the neurological function and the cerebral infarction area after cerebral infarction and involves a lower risk of hemorrhage.

## Claims

**1.** A compound of formula I or a pharmaceutically acceptable salt thereof,

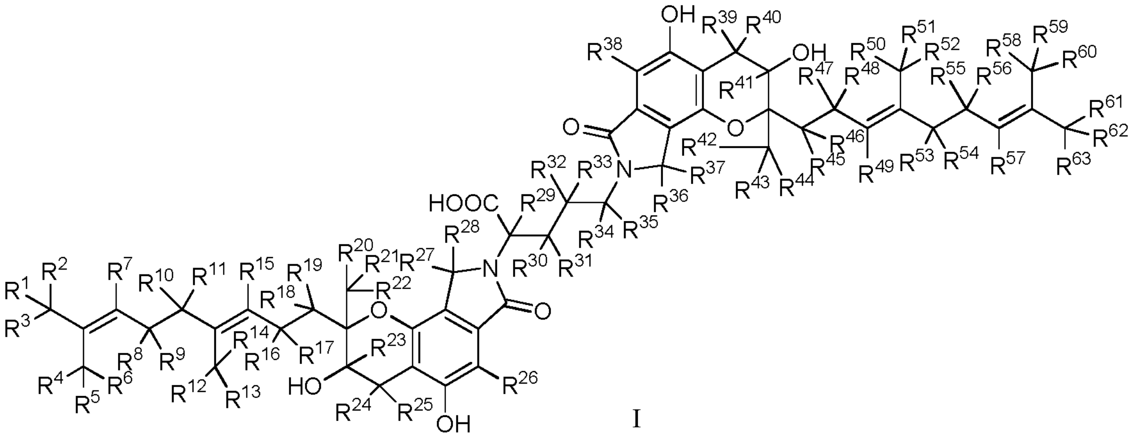

I

wherein $R^1$, $R^2$, $R^3$, $R^4$, $R^5$, $R^6$, $R^7$, $R^8$, $R^9$, $R^{10}$, $R^{11}$, $R^{12}$, $R^{13}$, $R^{14}$, $R^{15}$, $R^{16}$, $R^{17}$, $R^{18}$, $R^{19}$, $R^{20}$, $R^{21}$, $R^{22}$, $R^{23}$, $R^{24}$, $R^{25}$, $R^{26}$, $R^{27}$, $R^{28}$, $R^{29}$, $R^{30}$, $R^{31}$, $R^{32}$, $R^{33}$, $R^{34}$, $R^{35}$, $R^{36}$, $R^{37}$, $R^{38}$, $R^{39}$, $R^{40}$, $R^{41}$, $R^{42}$, $R^{43}$, $R^{44}$, $R^{45}$, $R^{46}$, $R^{47}$, $R^{48}$, $R^{49}$, $R^{50}$, $R^{51}$, $R^{52}$, $R^{53}$, $R^{54}$, $R^{55}$, $R^{56}$, $R^{57}$, $R^{58}$, $R^{59}$, $R^{60}$, $R^{61}$, $R^{62}$ and $R^{63}$ are each independently hydrogen or deuterium, and at least one of $R^1$, $R^2$, $R^3$, $R^4$, $R^3$, $R^6$, $R^7$, $R^8$, $R^9$, $R^{10}$, $R^{11}$, $R^{12}$, $R^{13}$, $R^{14}$, $R^{13}$, $R^{16}$, $R^{17}$, $R^{18}$, $R^{19}$, $R^{20}$, $R^{21}$, $R^{22}$, $R^{23}$, $R^{24}$, $R^{25}$, $R^{26}$, $R^{27}$, $R^{28}$, $R^{29}$, $R^{30}$, $R^{31}$, $R^{32}$, $R^{33}$, $R^{34}$, $R^{33}$, $R^{36}$, $R^{37}$, $R^{38}$, $R^{39}$, $R^{40}$, $R^{41}$, $R^{42}$, $R^{43}$, $R^{44}$, $R^{45}$, $R^{46}$, $R^{47}$, $R^{48}$, $R^{49}$, $R^{50}$, $R^{51}$, $R^{52}$, $R^{53}$, $R^{54}$, $R^{55}$, $R^{56}$, $R^{57}$, $R^{58}$, $R^{59}$, $R^{60}$, $R^{61}$, $R^{62}$ and $R^{63}$ is deuterium.

**2.** The compound or the pharmaceutically acceptable salt thereof according to claim 1, wherein $R^{27}$ is selected from deuterium; $R^{28}$ is selected from deuterium; $R^{36}$ is selected from deuterium; $R^{37}$ is selected from deuterium.

**3.** The compound or the pharmaceutically acceptable salt thereof according to claim 1 or 2, wherein $R^{26}$ is selected from deuterium; $R^{38}$ is selected from deuterium.

**4.** The compound or the pharmaceutically acceptable salt thereof according to any one of claims 1-3, wherein $R^{24}$ is selected from deuterium; $R^{25}$ is selected from deuterium; $R^{39}$ is selected from deuterium; $R^{40}$ is selected from deuterium.

**5.** The compound or the pharmaceutically acceptable salt thereof according to any one of claims 1-3, wherein $R^{29}$ is selected from deuterium.

**6.** The compound or the pharmaceutically acceptable salt thereof according to any one of claims 1-5, wherein $R^{30}$ is selected from deuterium; $R^{31}$ is selected from deuterium.

**7.** The compound or the pharmaceutically acceptable salt thereof according to any one of claims 1-6, wherein $R^{34}$ is selected from deuterium; $R^{35}$ is selected from deuterium.

**8.** The compound or the pharmaceutically acceptable salt thereof according to any one of claims 1-7, wherein $R^{16}$ is selected from deuterium; $R^{17}$ is selected from deuterium; $R^{47}$ is selected from deuterium; $R^{48}$ is selected from deuterium.

**9.** The compound or the pharmaceutically acceptable salt thereof according to any one of claims 1-8, wherein $R^{12}$ is selected from deuterium; $R^{13}$ is selected from deuterium; $R^{14}$ is selected from deuterium.

**10.** The compound or the pharmaceutically acceptable salt thereof according to any one of claims 1-9, wherein $R^{50}$ is selected from deuterium; $R^{51}$ is selected from deuterium; $R^{52}$ is selected from deuterium.

**11.** The compound or the pharmaceutically acceptable salt thereof according to any one of claims 1-10, wherein $R^{23}$ is selected from deuterium; $R^{41}$ is selected from deuterium.

**12.** The compound or the pharmaceutically acceptable salt thereof according to any one of claims 1-11, wherein $R^{32}$ is selected from deuterium; $R^{33}$ is selected from deuterium.

**13.** The compound or the pharmaceutically acceptable salt thereof according to any one of claims 1-5 and 12, wherein

$R^{30}$ is selected from deuterium; $R^{31}$ is selected from deuterium; $R^{32}$ is selected from deuterium; $R^{33}$ is selected from deuterium; $R^{34}$ is selected from deuterium; $R^{35}$ is selected from deuterium.

**14.** The compound or the pharmaceutically acceptable salt thereof according to any one of claims 1-13, wherein $R^1$ is selected from deuterium; $R^2$ is selected from deuterium; $R^3$ is selected from deuterium.

**15.** The compound or the pharmaceutically acceptable salt thereof according to any one of claims 1-14, wherein $R^4$ is selected from deuterium; $R^5$ is selected from deuterium; $R^6$ is selected from deuterium.

**16.** The compound or the pharmaceutically acceptable salt thereof according to any one of claims 1-15, wherein $R^{58}$ is selected from deuterium; $R^{59}$ is selected from deuterium; $R^{60}$ is selected from deuterium.

**17.** The compound or the pharmaceutically acceptable salt thereof according to any one of claims 1-16, wherein $R^{61}$ is selected from deuterium; $R^{62}$ is selected from deuterium; $R^{63}$ is selected from deuterium.

**18.** The compound or the pharmaceutically acceptable salt thereof according to claim 17, wherein $R^1$ is selected from deuterium; $R^2$ is selected from deuterium; $R^3$ is selected from deuterium; $R^4$ is selected from deuterium; $R^5$ is selected from deuterium; $R^6$ is selected from deuterium; $R^{58}$ is selected from deuterium; $R^{59}$ is selected from deuterium; $R^{60}$ is selected from deuterium; $R^{61}$ is selected from deuterium; $R^{62}$ is selected from deuterium; $R^{63}$ is selected from deuterium.

**19.** The compound or the pharmaceutically acceptable salt thereof according to any one of claims 1-18, wherein $R^7$ is selected from deuterium; $R^{57}$ is selected from deuterium.

**20.** The compound or the pharmaceutically acceptable salt thereof according to claim 1, being

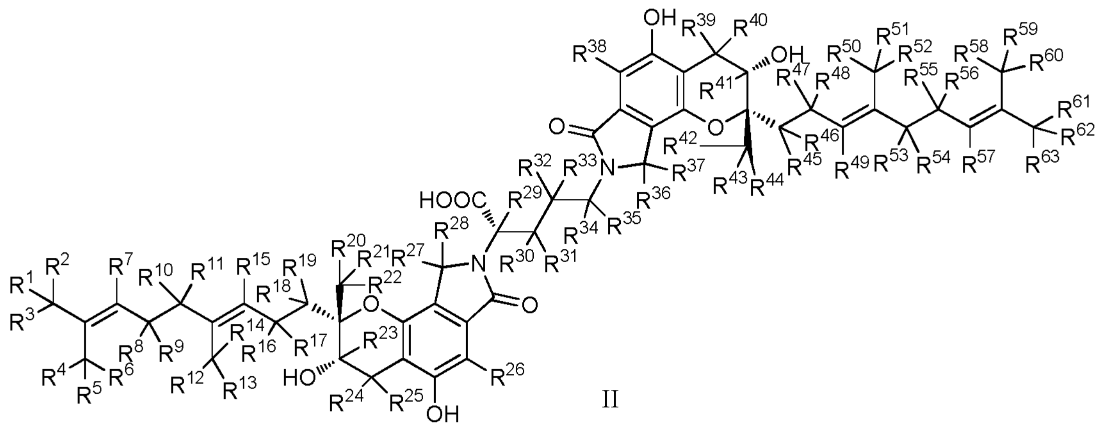

II

.

**21.** A compound of formula I or a pharmaceutically acceptable salt thereof, being selected from the group consisting of:

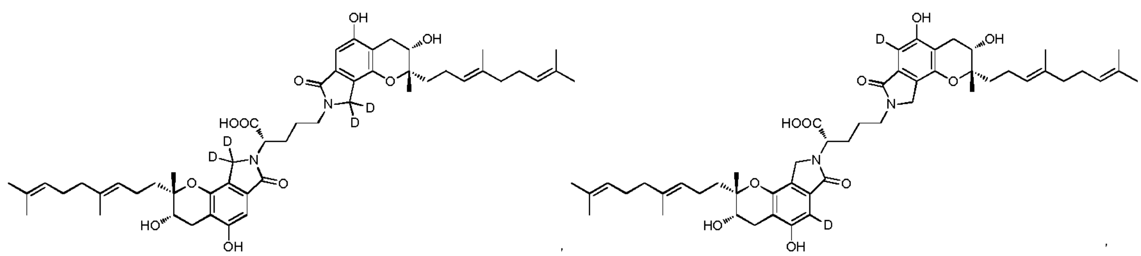

, ,

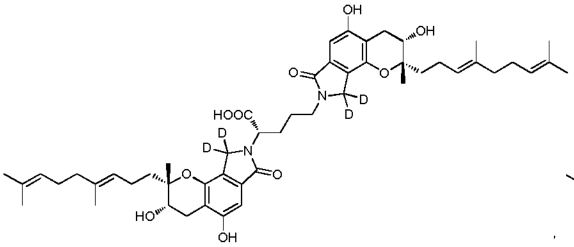
,
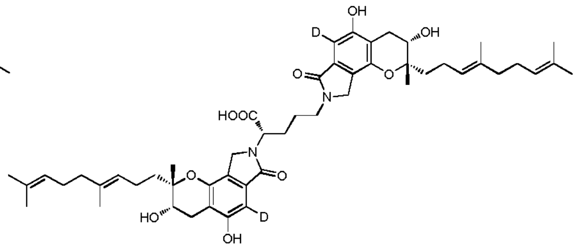
,
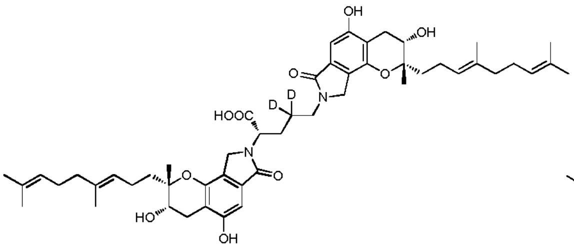
,
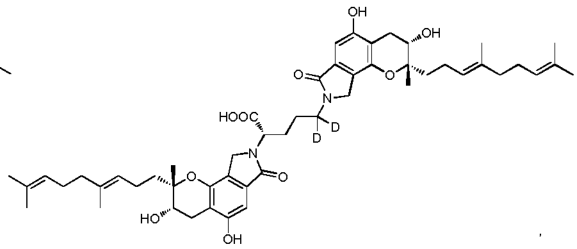
,
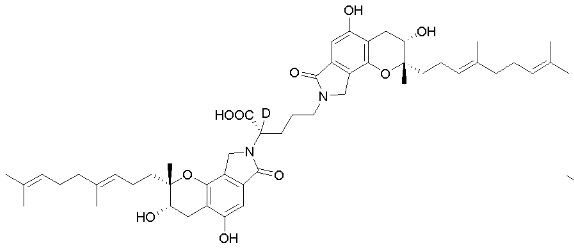
,
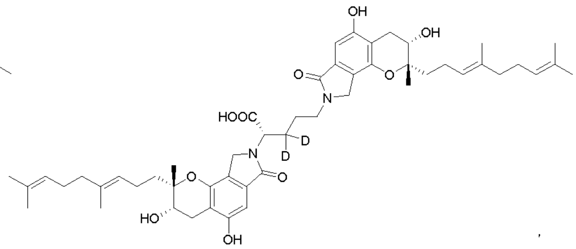
,
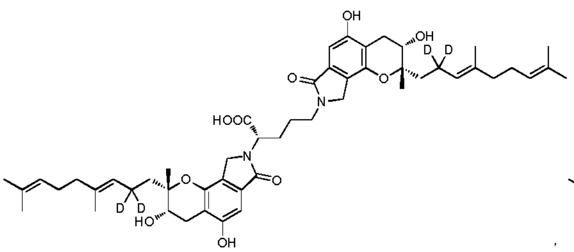
,
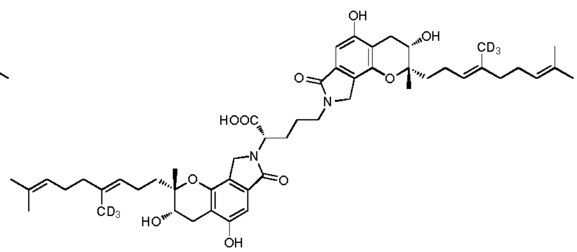
,
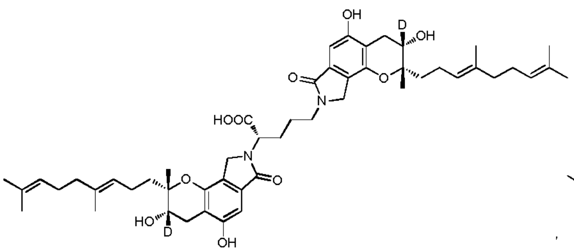
,
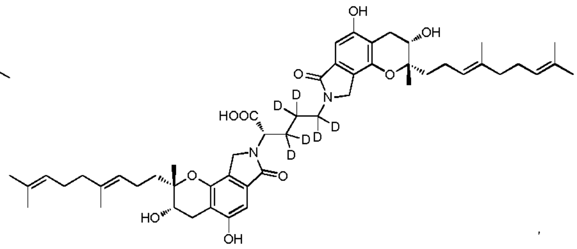
,
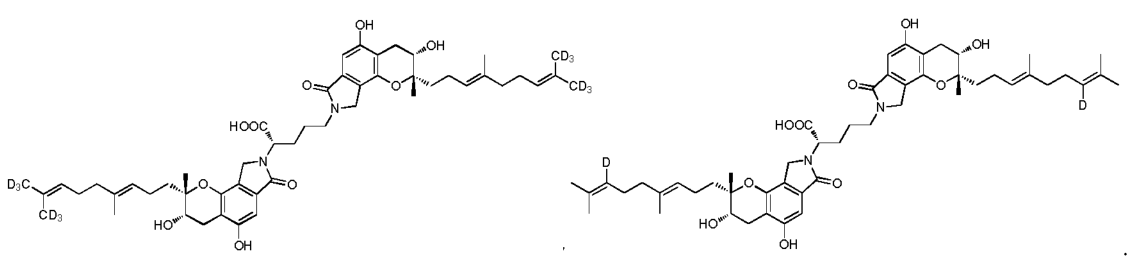
,
.

**22.** The compound or the salt thereof according to any one of claims 1-21, wherein deuterium atoms have an abundance that is at least 4000 times, preferably at least 5500 times, and more preferably at least 6000 times the natural abundance of deuterium.

**23.** A pharmaceutical composition comprising a therapeutically effective amount of at least one of the compound or the pharmaceutically acceptable salt thereof according to any one of claims 1-22 and a pharmaceutically acceptable excipient.

**24.** A method for preventing and/or treating a cardiovascular or cerebrovascular disease, comprising administering to a patient a therapeutically effective amount of the compound or the pharmaceutically acceptable salt thereof according to any one of claims 1-22 or the pharmaceutical composition according to claim 23, wherein the disease is preferably a thromboembolic disease, and more preferably myocardial infarction, angina pectoris, reocclusion and restenosis after angioplasty or aortic coronary artery shunt, disseminated intravascular coagulation, stroke, transient ischemic attack, peripheral arterial occlusive disease, pulmonary embolism or deep vein thrombosis.

**25.** Use of the compound or the pharmaceutically acceptable salt thereof according to any one of claims 1-22 or the pharmaceutical composition according to claim 23 in the preparation of a medicament for preventing and/or treating a cardiovascular or cerebrovascular disease, wherein the disease is preferably a thromboembolic disease, and more preferably myocardial infarction, angina pectoris, reocclusion and restenosis after angioplasty or aortic coronary artery shunt, disseminated intravascular coagulation, stroke, transient ischemic attack, peripheral arterial occlusive disease, pulmonary embolism or deep vein thrombosis.

## INTERNATIONAL SEARCH REPORT

International application No.
**PCT/CN2022/075740**

**A. CLASSIFICATION OF SUBJECT MATTER**

C07D 519/00(2006.01)i; A61K 31/407(2006.01)i; A61P 7/02(2006.01)i; A61P 9/10(2006.01)i

According to International Patent Classification (IPC) or to both national classification and IPC

**B. FIELDS SEARCHED**

Minimum documentation searched (classification system followed by classification symbols)

C07D A61K A61P

Documentation searched other than minimum documentation to the extent that such documents are included in the fields searched

Electronic data base consulted during the international search (name of data base and, where practicable, search terms used)

WPI, EPODOC, CNPAT, CNKI, CAPLUS(STN), REGISTRY(STN), MARPAT(STN), Baidu学术, BAIDU SCHOLAR; Google 学术; GOOGLE SCHOLAR: 上海森辉, 上海盛迪, 江苏恒瑞, 祝令建, 于秀招, 刘晓武, 贡培培, 陈磊, 孔祥林, 黄建, 孙飘扬, 栓塞, 溶栓, 梗塞, SMTP, thrombo? , 结构式检索, structural formula search

**C. DOCUMENTS CONSIDERED TO BE RELEVANT**

| Category* | Citation of document, with indication, where appropriate, of the relevant passages | Relevant to claim No. |
|---|---|---|
| A | CN 102115478 A (SHANGHAI OCEAN UNIVERSITY) 06 July 2011 (2011-07-06) claims 1 and 5-15 | 1-25 |
| A | CN 106946910 A (SHANGHAI OCEAN UNIVERSITY) 14 July 2017 (2017-07-14) claims 1-9 | 1-25 |
| A | SAWADA, H. et al. "SMTP-7, a novel small-molecule thrombolytic for ischemic stroke: a study in rodents and primates" *Journal of Cerebral Blood Flow & Metabolism,* Vol. 34, No. 2, 31 December 2014 (2014-12-31), ISSN: 0271-678X, pp. 235-241 | 1-25 |
| A | SHIBATA, K. et al. "A novel finding of a low-molecular-weight compound, SMTP-7, having thrombolytic and anti-inflammatory effects in cerebral infarction of mice" *Naunyn-Schmiedeberg's Archives of Pharmacology,* Vol. 382, No. 3, 03 August 2010 (2010-08-03), ISSN: 0028-1298, pp. 245-253 | 1-25 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **18 April 2022** | **06 May 2022** |

Name and mailing address of the ISA/CN

**China National Intellectual Property Administration (ISA/CN)**
**No. 6, Xitucheng Road, Jimenqiao, Haidian District, Beijing 100088, China**

Facsimile No. **(86-10)62019451**

Authorized officer

Telephone No.

**INTERNATIONAL SEARCH REPORT**

International application No.
**PCT/CN2022/075740**

**C. DOCUMENTS CONSIDERED TO BE RELEVANT**

| Category* | Citation of document, with indication, where appropriate, of the relevant passages | Relevant to claim No. |
|---|---|---|
| A | HU, W. M. et al. "SMTP (Stachybotrys microspora triprenyl phenol) enhances clot clearance in a pulmonary embolism model in rats" *Thrombosis Journal,* Vol. 10, No. 2, 09 January 2012 (2012-01-09), ISSN: 1477-9560, pp. 1-9 | 1-25 |
| A | WO 2020184691 A1 (SHOWA UNIVERSITY et al.) 17 September 2020 (2020-09-17) abstract, and table 5, compounds | 1-25 |
| A | WO 2012115209 A1 (NATIONAL UNIVERSITY CORPORATION TOKYO UNIVERSITY et al.) 30 August 2012 (2012-08-30) claims 1-7 and description, paragraph [0067] | 1-25 |

Form PCT/ISA/210 (second sheet) (January 2015)

**INTERNATIONAL SEARCH REPORT**

International application No.
**PCT/CN2022/075740**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **24**
because they relate to subject matter not required to be searched by this Authority, namely:

[1] Claim 24 relates to a method of treating a human/animal body, and the present search report is provided on the basis of a corresponding pharmaceutical use.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (January 2015)

**INTERNATIONAL SEARCH REPORT**
**Information on patent family members**

International application No.
**PCT/CN2022/075740**

| Patent document cited in search report | Publication date (day/month/year) | Patent family member(s) | Publication date (day/month/year) |
|---|---|---|---|
| CN 102115478 A | 06 July 2011 | None | |
| CN 106946910 A | 14 July 2017 | None | |
| WO 2020184691 A1 | 17 September 2020 | IL 286289 D0 | 31 October 2021 |
| | | EA 202192483 A1 | 24 December 2021 |
| | | EP 3939659 A1 | 19 January 2022 |
| | | KR 20220009371 A | 24 January 2022 |
| | | JP WO2020184691 A1 | 17 September 2020 |
| | | CA 3133204 A1 | 17 September 2020 |
| | | SG 11202109982 Y A | 28 October 2021 |
| | | AU 2020238177 A1 | 04 November 2021 |
| WO 2012115209 A1 | 30 August 2012 | JP WO2012115209 A1 | 07 July 2014 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **WEIMIN H ; SHIGEKI O et al.** *J. Antibiot.,* 2000, vol. 53 (3), 241-247
- *Synlett,* 2016, vol. 27 (2), 309-312
- *Journal of the American Chemical Society,* 2017, vol. 139 (39), 13830-13836
- *Journal of the American Chemical Society,* 2018, vol. 140 (23), 7116-7126
- *J Cereb Blood Flow Metab,* 1997, vol. 17 (2), 123-135